# EUROPÄISCHE PATENTANMELDUNG

(11) **EP 3 459 959 A1**
(43) Veröffentlichungstag der Anmeldung: **27.03.2019**
(21) Anmeldenummer: 17193162.9
(22) Anmeldetag: 26.09.2017
(51) Int. Cl.: C07F 9/6571, C07C 45/50

(54) **BISPHOSPHITLIGANDEN MIT BUTANOAT-ZENTRALBAUSTEIN**

(71) Anmelder: Evonik Degussa GmbH, 45128 Essen (DE)
(72) Erfinder: ZHANG, Baoxin, 18106 Rostock (DE); BÖRNER, Armin, 18059 Rostock (DE); SELENT, Detlef, 18059 Rostock (DE); FRANKE, Robert, 45772 Marl (DE)

(57) **Zusammenfassung**

Bisphosphitliganden mit Butanoat-Zentralbaustein und deren Verwendung als Liganden in der Hydroformylierung.

## Beschreibung

Die Erfindung betrifft Bisphosphitliganden mit Butanoat-Zentralbaustein und deren Verwendung als Liganden in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Der Erfindung lag die Aufgabe zugrunde, Bisphosphitliganden bereitzustellen, welche als Liganden in der Hydroformylierung eingesetzt werden können, und dort eine gute Ausbeute an n-Aldehyd liefern.

Gelöst wird die Aufgabe durch eine Verbindung gemäß Anspruch 1.

Verbindung der Formel (**I**): 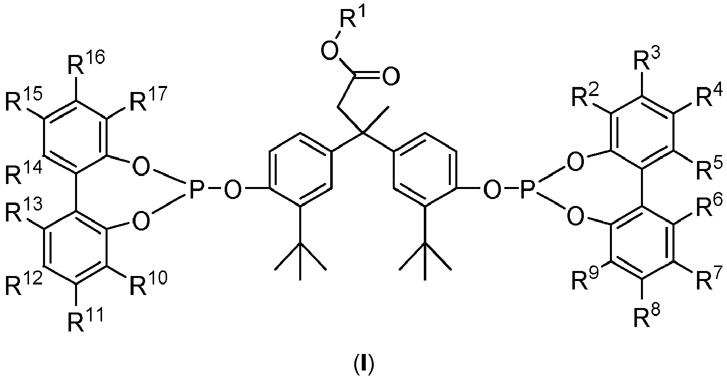 wobei
R¹ für -(C₁-C₁₂)-Alkyl steht;
R², R³, R⁴, R⁵, R⁶, R⁷, R⁸, R⁹, R¹⁰, R¹¹, R¹² , R¹³ R¹⁴ R¹⁵ R¹⁶, R¹⁷ ausgewählt sind aus: -H, - (C₁-C₁₂)-Alkyl, -O-(C₁-C₁₂)-Alkyl, -(C₆-C₂₀)-Aryl, -O-(C₆-C₂₀)-Aryl, Halogen,
wobei die genannten Alkylgruppen und Arylgruppen wie folgt substituiert sein können: -(C₁-C₁₂)-Alkyl, -O-(C₁-C₁₂)-Alkyl, Halogen;
und mindestens einer der Reste R², R³, R⁴, R⁵, R⁶, R⁷, R⁸, R⁹, R¹⁰, R¹¹, R¹², R¹³, R¹⁴, R¹⁵, R¹⁶, R¹⁷ nicht für -H steht.

In einer Ausführungsform steht R¹ für -CH₃.

In einer Ausführungsform stehen R², R⁴, R⁷, R⁹, R¹⁰, R¹², R¹⁵, R¹⁷ für -(C₁-C₁₂)-Alkyl.

In einer Ausführungsform stehen R², R⁴, R⁷, R⁹, R¹⁰, R¹², R¹⁵, R¹⁷ für *-tert-*Bu*.*

In einer Ausführungsform stehen R², R⁴, R⁷, R⁹, R¹⁰, R¹², R¹⁵, R¹⁷ für den gleichen Rest.

In einer Ausführungsform stehen R³ R⁵, R⁶, R⁸, R¹¹, R¹³, R¹⁴, R¹⁶ für -H.

In einer Ausführungsform stehen R³, R⁵, R⁶, R⁸, R¹¹, R¹³, R¹⁴, R¹⁶ für den gleichen Rest.

In einer Ausführungsform weist die Verbindung die Struktur (**1**) auf:

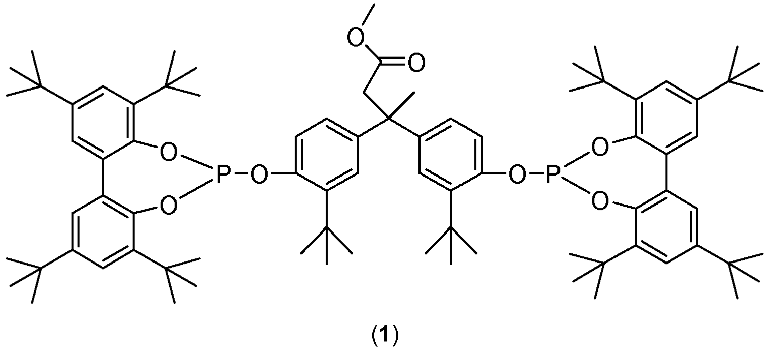

Neben der Verbindung als Stoff wird auch dessen Verwendung als Ligand in einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird auch ein Verfahren beansprucht, in welchem eine zuvor beschriebene Verbindung eingesetzt wird.

Verfahren umfassend die Verfahrensschritte:
a) Vorlegen eines Olefins,
b) Zugabe eines Komplexes, welcher eine zuvor beschriebene Verbindung umfasst, sowie ein Metall ausgewählt aus: Rh, Ru, Co, Ir,
   oder einer zuvor beschriebene Verbindung und einer Substanz, welche ein Metall ausgewählt aus: Rh, Ru, Co, Ir aufweist;
c) Zuführen von H₂ und CO,
d) Erwärmen des Reaktionsgemisches, wobei das Olefin zu einem Aldehyd umgesetzt wird.

In einer Variante des Verfahrens ist das Metall Rh.

Im Folgenden wird die Erfindung anhand von Ausführungsbeispielen näher erläutert.

### Ligandensynthese

### Ligand (1)

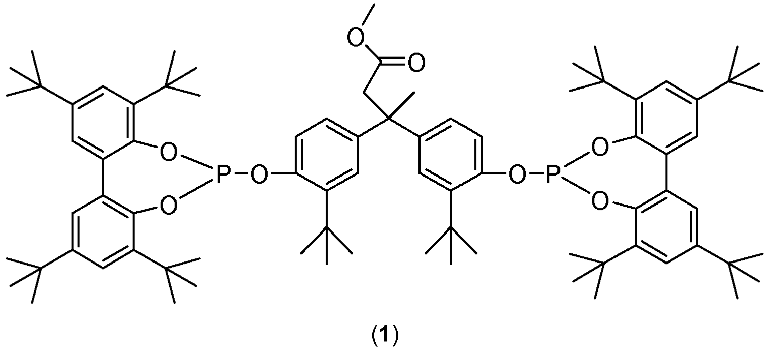

Eine gerührte Lösung von 2,4,8,10-Tetra-*tert*-butyl-6-chlordibenzo[*d*,*f*][1,3,2]-dioxaphosphepin (2,08 g; 4,39 mmol) in THF (12 ml) wird bei 0 °C tropfenweise mit einer Mischung aus Methyl 3,3-bis(3-(*tert*-butyl)-4-hydroxyphenyl)butanoate (0,69 g; 1,73 mmol), Triethylamin (1,3 ml) und THF (12 ml) versetzt. Man lässt über Nacht bei Raumtemperatur rühren, filtriert, engt das Filtrat im Vakuum ein und trocknet den erhaltenen Rückstand 2 h bei 50°C / 0,1 mbar. Die säulenchromatografische Reinigung (n-Heptan/Dichlormethan = 2/1, R_{f} = 0,27) ergibt 1,45 g (1,14 mmol; 65,7%) des Biphosphites.
Analyse: ³¹P-NMR (CD₂Cl₂): 140,8 (s) ppm.
¹H-NMR (CD₂Cl₂): 1,16 (18H); 1,36 (36H); 1,39 (36H); 1,83 (3H); 3,04 (2H); 3,41 (3H); 6,93-7,06 (6H); 7,23 (d, 4H); 7,44 (d, 4H) ppm.
ESI-TOF HRMS: *m*/*e* 1275,79046 (M+H)⁺.

### Ligand (2) (Vergleichsligand)

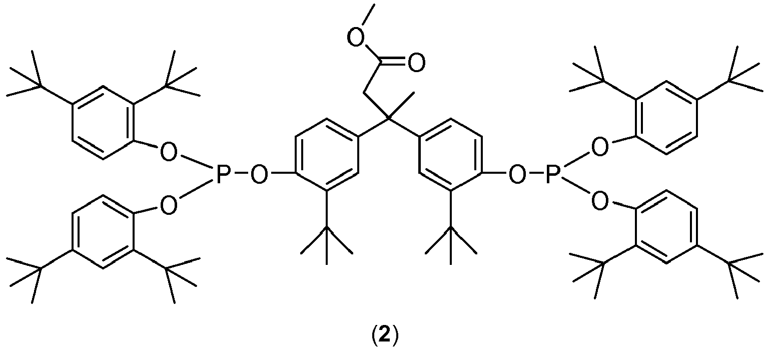

Synthese von Methyl 3,3-bis(3-(tert-butyl)-4-((dichlorophosphino)oxy)phenyl)butanoate: Eine Lösung von Phosphortrichlorid (0,42 ml; 4,81 mmol) in THF (3 ml) wird bei 0 °C unter Rühren tropfenweise mit einer Mischung aus Methyl 3,3-bis(3-(*tert*-butyl)-4-hydroxyphenyl)butanoate (0,77 g; 1,93 mmol), Triethylamin (1,12 ml) und THF (30 ml) versetzt. Man lässt über Nacht bei Raumtemperatur rühren, filtriert, engt das Filtrat im Vakuum ein und trocknet den erhaltenen Rückstand 2 h bei 50°C / 0,1 mbar. Das Produkt wird in Toluol (15 ml) gelöst, die Lösung über eine G4-Fritte filtriert und erneut zur Trockne eingeengt. Ausbeute: 0,751 g (1,25 mmol; 64,8%). Das so erhaltene Produkt wurde direkt weiterverwendet.
Analyse: ³¹P-NMR (CD₂Cl₂): 185,4 ppm. ¹H-NMR (CD₂Cl₂): 1,33 (18H); 1,87 (3H); 3,10 (2H); 3,44 (3H); 7,02-7,41 (6H) ppm.

Eine gerührte Mischung aus 2,4-Di-*tert*-butylphenol (1,65 g; 8,01 mmol), Triethylamin (1,7 ml) und THF (10 ml) wird bei 0 °C tropfenweise mit einer Lösung von Methyl-3,3-bis(3-(*tert*-butyl)-4-((dichlorphosphanyl)oxy)phenyl)butanoate (1,00 g; 1,67 mmol) in THF (16 ml) versetzt. Man läßt über Nacht bei Raumtemperatur rühren, filtriert, engt das Filtrat im Vakuum ein und trocknet den erhaltenen Rückstand 2 h bei 50°C / 0,1 mbar. Die säulenchromatografische Reinigung (n-Heptan/Dichlormethan = 1/1, R_{f} = 0,53) ergibt 0,40 g (0,316 mmol; 18,9%) des Biphosphites.
Analyse: ³¹P-NMR (CD₂Cl₂): 130,3 (s) ppm.
¹H-NMR (CD₂Cl₂): 1,28 (36H); 1,30 (18H); 1,37 (36H); 1,81 (3H); 3,04 (2H); 3,38 (3H); 6,91-7,38 (18H) ppm.
ESI-TOF HRMS: *m*/*e* 1279,82104 (M+H)⁺.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzte Gemisch der n-Octene (Oxeno GmbH, Octenisomerengemisch aus 1-Octen: 3 %; *cis*+*trans-*2*-*Octen: 49 %; *cis*+*trans-*3-Octen: 29 %; *cis*+*trans-*Octen-4: 16 %; gerüstisomere Octene: 3 %) wurden über Natrium am Rückfluß erhitzt und unter Argon destilliert.

Im Autoklaven wurden unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Von der Rhodiumverbindung wurden 10 ml einer 4,31 millimolaren Lösung in den Autoklaven gegeben. Anschließend wurde die entsprechende Menge an Liganden (P/Rh = 10:1) in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurden die n-Octene (10,70 g; 95,35 mmol) eingefüllt. Der Autoklav wurde bei einem Gesamtgasdruck (Synthesegas: Linde; H₂ (99,999%) : CO (99,997%) = 1:1) von 42 bar aufgeheizt. Nach Erreichen der Reaktionstemperatur von 120 °C wurde der Synthesegasdruck auf 48,5 bar erhöht und das Olefin mit einem in der Druckpipette eingestellten Überdruck von ca. 3 bar in den Autoklaven gepresst. Die Reaktion wurde bei konstantem Druck von 50 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

### Ergebnisse der Katalyseversuche

**Tabelle: Hydroformylierung der n-Octene**

| Ligand | Ausbeute n-Aldehyd (%) |
|---|---|
| **1*** | 20,1 |
| **2** | 18,4 |

| | |
|---|---|
| * erfindungsgemäße Verbindung | |

Wie die oben beschriebenen Versuche zeigen, wird die Aufgabe durch eine erfindungsgemäße Verbindung gelöst.

## Patentansprüche

1. Verbindung der Formel (**I**): 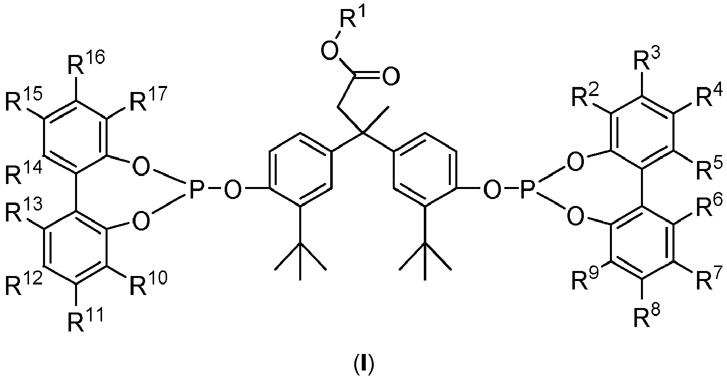 wobei
R¹ für -(C₁-C₁₂)-Alkyl steht;
R², R³, R⁴, R⁵, R⁶, R⁷, R⁸, R⁹, R¹⁰, R¹¹, R¹² , R¹³ R¹⁴ R¹⁵ R¹⁶, R¹⁷ ausgewählt sind aus: -H, - (C₁-C₁₂)-Alkyl, -O-(C₁-C₁₂)-Alkyl, -(C₆-C₂₀)-Aryl, -O-(C₆-C₂₀)-Aryl, Halogen,
wobei die genannten Alkylgruppen und Arylgruppen wie folgt substituiert sein können: -(C₁-C₁₂)-Alkyl, -O-(C₁-C₁₂)-Alkyl, Halogen;
und mindestens einer der Reste R², R³, R⁴, R⁵, R⁶, R⁷, R⁸, R⁹, R¹⁰, R¹¹, R¹², R¹³, R¹⁴, R¹⁵, R¹⁶, R¹⁷ nicht für -H steht.

2. Verbindung nach Anspruch 1,
wobei R¹ für -CH₃ steht.

3. Verbindung nach einem der Ansprüche 1 oder 2,
wobei R², R⁴, R⁷, R⁹, R¹⁰, R¹², R¹⁵, R¹⁷ für -(C₁-C₁₂)-Alkyl stehen.

4. Verbindung nach einem der Ansprüche 1 bis 3,
wobei R², R⁴, R⁷, R⁹, R¹⁰, R¹² , R¹⁵, R¹⁷ für *-tert-*Bu stehen.

5. Verbindung nach einem der Ansprüche 1 bis 4,
wobei R², R⁴, R⁷, R⁹, R¹⁰, R¹² , R¹⁵, R¹⁷ für den gleichen Rest stehen.

6. Verbindung nach einem der Ansprüche 1 bis 5,
wobei R³, R⁵, R⁶, R⁸, R¹¹, R¹³, R¹⁴, R¹⁶ für -H stehen.

7. Verbindung nach einem der Ansprüche 1 bis 6,
wobei R³, R⁵, R⁶, R⁸, R¹¹, R¹³, R¹⁴, R¹⁶ für den gleichen Rest stehen.

8. Verbindung nach einem der Ansprüche 1 bis 7,
welche die Struktur (**1**) aufweist:

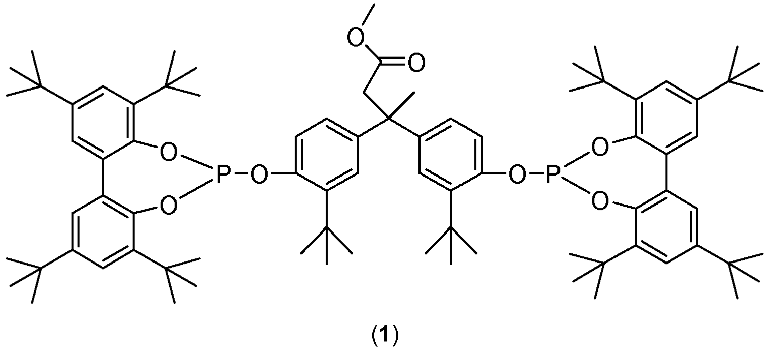

9. Verwendung einer Verbindung nach Anspruch 1 bis 8,
in einem Ligand-Metall-Komplex zur Katalyse einer Hydroformylierungsreaktion.

10. Verfahren umfassend die Verfahrensschritte:
a) Vorlegen eines Olefins,
b) Zugabe eines Komplexes, welcher eine Verbindung gemäß den Ansprüchen 1 bis 8 umfasst, sowie ein Metall ausgewählt aus: Rh, Ru, Co, Ir,
oder einer Verbindung nach einem der Ansprüche 1 bis 8 und einer Substanz, welche ein Metall ausgewählt aus: Rh, Ru, Co, Ir aufweist;
c) Zuführen von H₂ und CO,
d) Erwärmen des Reaktionsgemisches, wobei das Olefin zu einem Aldehyd umgesetzt wird.
